# EUROPEAN PATENT SPECIFICATION

(11) **EP 3 740 125 B1**
(45) Date of publication and mention of the grant of the patent: **15.11.2023**
(21) Application number: 19704849.9
(22) Date of filing: 15.01.2019
(51) Int. Cl.: A61B 5/024, A61B 5/01

(54) **METHOD, APPARATUS, AND SYSTEM FOR DETECTING STAGES OF SLEEP OF PERSON**
VERFAHREN, VORRICHTUNG UND SYSTEM ZUR ERKENNUNG VON SCHLAFPHASEN EINER PERSON
PROCÉDÉ, APPAREIL ET SYSTÈME DE DÉTECTION DE STADES DE SOMMEIL D'UNE PERSONNE

(30) Priority: 19.01.2018 FI 20185051
(43) Date of publication of application: 25.11.2020
(73) Proprietor: Night Train Oy, 90420 Oulu (FI)
(72) Inventor: RYTKY, Juha, 90420 Oulu (FI); MYLLYOJA, Kimmo, 90520 Oulu (FI); PYLKKÖ, Heikki, 54800 Savitaipale (FI)
(74) Representative: LEITZINGER OY
(86) International application number: PCT/FI2019/050030
(87) International publication number: WO 2019/141904

(56) References cited:
- EP-A1- 3 199 197
- CN-A- 104 095 615
- US-A1- 2005 190 065
- US-A1- 2013 245 465
- US-A1- 2014 088 378
- US-A1- 2015 190 086

## Description

The invention relates to monitoring sleep of a person, and in particular, to detecting stages of sleep of the person.

### BACKGROUND INFORMATION

Sleep of a human follows a circadian rhythm, i.e. a sleep-wakefulness cycle that is on average 24.09 ± 0.2 h (24 h 5 min ± 12 min) for women and 24.19 ± 0.2 h (24 h 11 min ± 12 min) for men, aged 18 to 74 years. The circadian rhythm is typically synchronized to the day-night cycle, i.e. the light-darkness cycle. During sleep within the circadian rhythm, a natural sleep cycle repeats itself at a constant interval reflecting the 1-to-2-hour ultradian basic rest-activity cycle. The natural sleep cycle comprises a plurality of stages that repeat during the sleep. These stages include REM (Rapid Eye Movement) sleep and non-REM (NREM) sleep, which can be further divided in to light sleep and deep sleep.

An adult person may be considered to need on average a minimum of 4 full sleep cycles that are guided by the human circadian system controlling and the sleep-wake cycle. Less sleep may result in various negative effects to wellbeing and health. In order to ensure sufficient amount of sleep for a person, it may be desirable to try to monitor the sleep and to estimate the progress of the person's natural sleep cycles.

Various attempts have been made to estimate the progress of the natural sleep cycles, based on the nocturnal movements of a person, for example. However, there is no scientific evidence that the movements correlate with the sleep stages within the sleep cycle. Therefore, measurement of nocturnal movements of a person is not a reliable information source for tracking the sleep stages of the person.

EP-3199197-A1, US-2014/088378-A1, US-2013/245465-A1, US-2005/190065-A1, CN-104095615-A and US-2015/190086-A1 describe known methods for sleep stage evaluation based on temperature and heart rate measurements.

### BRIEF DISCLOSURE

An object of the present disclosure is to provide a method and an apparatus for implementing the method so as to alleviate the above disadvantages. The object of the disclosure is achieved by a method and apparatus which are characterized by what is stated in the independent claims. The preferred embodiments of the disclosure are disclosed in the dependent claims.

The present disclosure describes a method for detecting stages of sleep of a person. During REM (Rapid Eye Movement) sleep stage and deep sleep stage of a person's sleep, the person's body temperature control effectively shuts off and distal skin temperature changes on the limbs may be within just about 0,2 degrees Celsius. During the other sleep stages the human's temperature control is active and the distal skin temperature changes are much larger.

These two stages of sleep are therefore distinguishable from other stages of sleep and arousal (i.e. state of being awoken) of the person. However, it is difficult to distinguish these two stages from each other based on the temperature.

Heart rate variability (HRV) is another parameter that is affected by different stages of sleep. Importantly, REM sleep and deep sleep show different HRV characteristics. Therefore, it is possible to use HRV for distinguishing REM sleep and deep sleep from each other. By combining these two measurements (temperature variability and HRV), different stages of sleep can be identified.

The method according to the present disclosure provides accurate and cost-efficient means for detecting different stages of sleep. The measurements can be performed in a non-invasive manner that is more comfortable to the user. The method can be used for obtaining important information (e.g. ratios of different stages of sleep, length of sleep cycle, and circadian rhythm) on the sleep of the user.

### BRIEF DESCRIPTION OF THE DRAWINGS

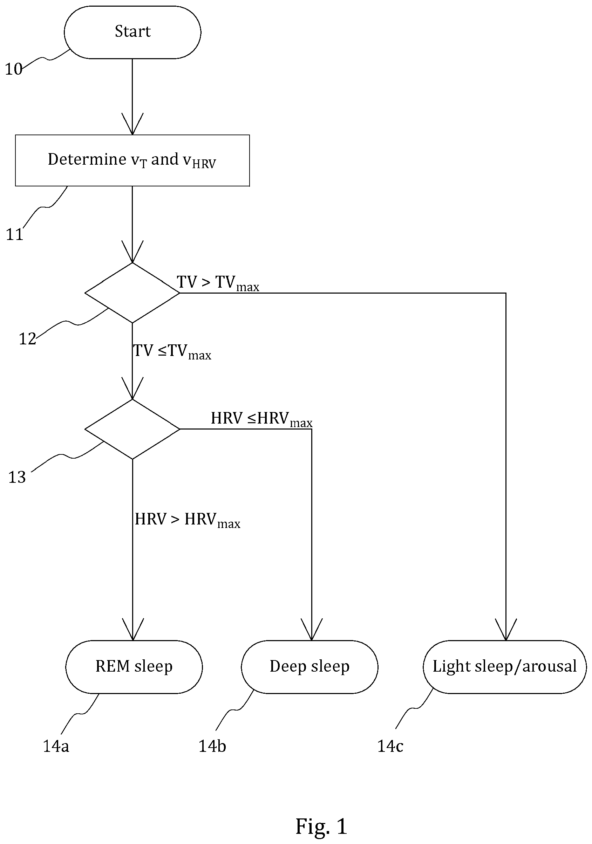

In the following the invention will be described in greater detail by means of preferred embodiments with reference to the attached drawings, in which
Figure 1 shows a simplified diagram of an exemplary embodiment of the method according to the present disclosure.

### DETAILED DISCLOSURE

The present disclosure describes a method for detecting stages of sleep of a person. A method according to the present disclosure comprises receiving temperature data and heart rate data of the person, determining temperature variability on the basis of the temperature data, determining heart rate variability (HRV) on the basis of the heart rate data, and detecting stages of sleep of the person on the basis of the temperature variability and heart rate variability. In the context of the present disclosure, the term "variability" represents dispersion (scatter, spread) of the measurements over time. The variability may be calculated as variance, standard deviation, and interquartile range of the measurements, for example.

The method may detect that the person is in a REM (Rapid Eye Movement) sleep stage when the temperature variability is below a first temperature variability limit and the heart rate variability is above a first heart rate variability limit. In other words, the method may comprise comparing the temperature variability with the first temperature variability limit, comparing the heart rate variability with the first heart rate variability limit, and considering that the person is in the REM sleep stage when a first condition is detected, the first condition being that the temperature variability is below the first temperature variability limit and the heart rate variability is above the first heart rate variability limit.

In addition or alternatively, the method may detect that the person is in a deep sleep stage when the temperature variability is below a second temperature variability limit and the heart rate variability is below a second heart rate variability limit. In other words, the method may comprise comparing the temperature variability with the second temperature variability limit, comparing the heart rate variability with the second heart rate variability limit, and considering that the person is in the deep sleep stage when a second condition is detected, the second condition being that the temperature variability is below the second temperature variability limit and the heart rate variability is below the second heart rate variability limit.

Figure 1 shows a simplified diagram of an exemplary embodiment of the method according to the present disclosure. In Figure 1, the first temperature variability limit is the same as the second temperature variability limit, and the first heart rate variability limit is the same as the second variability limit.

The method in Figure 1 starts from step initial step 10 from which the method moves to step 11. In step 11, the temperature variability TV and the heart rate variability HRV are determined and the method moves to step 12.

In step 12, the temperature variability TV is compared with a predetermined temperature variability limit TVₘₐₓ. If temperature variability TV exceeds the temperature variability limit TVₘₐₓ, the stage of sleep is considered to be light sleep stage or arousal and the method continues to a final step 14c identifying the sleep stage as light sleep or arousal. However, if temperature variability TV the same or less than temperature variability limit TVₘₐₓ, the method moves to step 13.

In step 13, the heart rate variability HRV is compared with a predetermined heart rate variability limit HRVₘₐₓ. If heart rate variability HRV exceeds the heart rate variability limit HRVₘₐₓ, the stage of sleep is considered to be REM sleep and the method continues to a final step 14a identifying the sleep stage as REM sleep. However, if heart rate variability HRV the same or less than the heart rate variability limit HRVₘₐₓ, the stage of sleep is considered to be REM sleep and the method continues to a final step 14b identifying the sleep stage as deep sleep.

A person has typically several brief periods of arousals during his/her nightly sleep. During these periods, the person is typically more restless, which can be detected as more physical activity. Therefore, in some embodiment, the method may further comprise measurement of physical activity (i.e. actigraphy). The measurement of physical activity of the person may be estimated based on an acceleration sensor attached to the person, for example. The measurement of physical activity can then be used for distinguishing the periods of arousal from the cycles of sleep. For example, in step 14c of Figure 1, arousal may be distinguished from light sleep based on the physical activity. The level of physical activity may be is compared with a predetermined physical activity limit, and if the level exceeds the limit, the stage of sleep may be considered to be arousal. If the level of physical activity does not exceed the limit, the stage of sleep may be considered to be light sleep.

Although Figure 1 uses only one limit for temperature variability and one limit for heart rate variability, the method according to the present disclosure may also utilize different temperature variability limits for REM sleep, deep sleep and light sleep/arousal. Alternatively or in addition, the method according to the present disclosure may also utilize different heart rate variability limits for REM sleep, deep sleep and light sleep/arousal.

With the method according to the present disclosure, important information on the quality of the sleep of a person can be obtained. Based on the detected stages of sleep, estimates of ratios of different stages of sleep during night can be calculated, for example. The ratios may be represented as percentages or amount (e.g. in minutes) of total sleep during the night, for example.

Another important parameter of sleep of a person is the interval (i.e. length) of a natural sleep cycle of the person. Typically, the interval (i.e. length) of a natural sleep cycle is approximately 90 minutes for an adult, but it may vary between individuals. For example, depending on the age of the person, the length of the sleep cycle may vary between 40 minutes to 130 minutes. However, within a time frame of a day or several days, the interval at which the natural sleep cycle repeats itself may be considered to remain rather constant. An adult person may be considered to need 4 to 6 full sleep cycles.

With the method according to the present disclosure, the interval between the occurrences of (or starts of) sleep cycles of specific type (e.g. REM sleep cycles or deep sleep cycles) can be detected. By determining the interval between these the sleep cycles, an estimate of the length of the natural sleep cycle of the person can be calculated. For example, a good estimate of the length of the sleep cycle may be calculated on the basis of an interval between the starts of the second and third REM sleep cycle after beginning of sleep. The estimate of the length of the sleep cycle may be based on an average of intervals between subsequent sleep cycles of the same type, for example.

Information on the length and contents of the natural sleep cycle can be coupled with the circadian rhythm, and in particular with melatonin onset (start of nocturnal melatonin production). There is a direct link between the melatonin onset and the circadian rhythm. The ability to fall asleep is linked to the melatonin onset. Thus, the melatonin onset provides a fixed reference point within the circadian rhythm of the person. The melatonin onset is observable as a change in the body temperature of the person. An estimate of the melatonin onset can be detected with a distal skin temperature measurement and an actigraphy measurement, for example. At the beginning of sleep of a person, his/her distal skin temperature rises rapidly for a while. However, at the same time the person typically remains relatively still which can be detected as a low actigraph value, e.g. in the form of low value (e.g. a value below a predetermined limit) of measurement data from an acceleration sensor.

Within the context of the present disclosure, temperature of a person may be measured in various ways. According to the invention the temperature samples originate from a temperature sensor arranged to measure the distal skin temperature. In this context, distal skin temperature refers to skin temperature at extremities of body, such as the limbs and the head of a person. For example, distal skin temperature may be measured from a finger, wrist, ankle, forehead or ear lobe. The distal skin temperature may be measured with a MEMS-based temperature sensor or an infrared sensor, for example. Further, an estimate of the heart rate may be extracted from graphical data provided by a digital video camera recording video of a portion of person's skin. Based on this estimate, variability of the relative temperature may be calculated, and changes in the variability of the relative temperature may then be used to detect the different stages of sleep and the melatonin onset.

The temperature sensor may be a part of a wearable device, for example. The temperature sensor may also be integrated to a vehicle, e.g. in the form of a video camera recording a view on the face of the driver of a car, and the method according to the present disclosure may be used for estimating the driver's alertness.

In order to be able to detect changes in the variability of the temperature, the temperature has to be sampled at a sufficient frequency. For example, the distal temperature may be sampled at least once a minute, e.g. once every 30 seconds. The temperature may be periodically sampled and the samples may be stored. Samples may be stored for one night or over a longer period, ranging from a plurality of days to a plurality of weeks, for example. Based on the samples, variability of the temperature may then be calculated by using known algorithms, for example. In case of longer measurement periods and larger quantities of data, the data may be sent from a wearable device making the measurements to a larger data storage, such as a computer server or a computing cloud, for example.

Information on detected stages of sleep may be utilized in various ways. For example, in a first embodiment of a method according to the present disclosure, the method comprises calculating ratios of different stages of sleep of the person based on the basis of the detected stages of sleep. This kind of information can provide an important insight to the quality of the sleep. The quality of sleep can have a significant effect on the wellbeing and health of a person. Thus, the method may be configured to provide information of ratios of different stages of sleep to the user. This information may then be utilized in estimating the quality of the sleep of the user, for example.

The detected stages may also be used for determining an optimal time window for sleep. In a second embodiment of a method according to the present disclosure, the method may comprise estimating an optimal instant for a wake-up for a person on the basis of the detected stages of sleep. If a wake-up (e.g. in the form of an alarm) is scheduled to occur at a light sleep stage within the natural sleep cycle, the person wakes up feeling fresh and energetic. Calculation of an optimal wake-up instant may comprise determining the length of a sleep cycle of the person, calculating the number of specific sleep stages already observed during night's sleep on the basis of the detected stages of sleep, and determining optimal wake-up instant on the basis of the length of the sleep cycle and the number of sleep stages already observed. For example, one or more lengths of the sleep cycle may be added to the last observed sleep stage of a specific type in order to determine an optimal wake-up instant so that the person will have slept a desired number (e.g. 4, 5, or 6) of sleep cycles at the optimal wake-up instant. Once the optimal instant has been reached, an alarm may be raised in order to wake up the person. In embodiments where the physical activity of the person is measured, an increased physical activity may be used as an indication of waking up from the sleep. For example, a detected increase the physical activity that is above a predetermined detection limit for a set amount of time may be used as an indicator that the alarm has performed its task.

In addition or alternatively in the second embodiment, estimates of optimal instants of wake-up may also be calculated beforehand on the basis of the length of the natural sleep cycle and a reference point within the circadian rhythm of the person, such as the melatonin onset. Thus, the method may comprise determining a length of a sleep cycle of the person, determining a fixed reference point within a circadian rhythm of the person, and determining the optimal wake-up instant on the basis of the length of the sleep cycle and the fixed reference point so that the person will have slept a desired number of sleep cycles at the optimal wake-up instant. For example, an optimal wake-up instant may be calculated by adding a plurality (e.g. 4, 5, or 6) of determined lengths of the natural sleep cycle to the time of a known time instant of the melatonin onset. Once the optimal instant has been reached, an alarm may be raised in order to wake up the person.

In addition or alternatively, the method according to the present disclosure may also be used for determining an estimate of an optimal instant for turning in for sleep. According to a third embodiment of the method according to the present disclosure, the method may determine the optimal sleep onset (i.e. an optimal instant for turning in for sleep) based on a reference point within the circadian rhythm of the person. The reference point may represent the melatonin onset, for example. The method may calculate the next optimal instant for a sleep onset based on the length of the sleep cycle, in case the person misses any preceding optimal instants for sleep onset.

In embodiments where distal skin temperature is measured, detection of a specific temperature change pattern within distal skin temperature data may be utilized in detecting the melatonin onset. A rapid, distinct pattern in the distal skin temperature can be observed after the nocturnal melatonin production has started. The temperature change pattern that is in the form of a drop in the distal skin temperature followed an increase in the distal skin temperature, wherein the drop and the increase occur within a time window of ten minutes or less, for example five minutes. The magnitude of the increase is higher than the magnitude of the drop. The magnitude of the drop may be approximately 0.5 °C and the magnitude of the increase may be approximately 1.5 °C, for example. When the characteristic temperature change pattern is observed in the distal temperature of a person, he or she has approximately 20 to 30 minutes to get to sleep. Thus, the temperature change pattern serves as a fixed reference point within the circadian rhythm. Once a person has fallen asleep, the temperature change patterns cease to occur. The temperature change patterns will re-start again if the person awakes in the middle of the night. This may be used as a further indicator of sleep when monitoring the sleep of a person. When she or he falls asleep, the distal skin temperature starts to increase constantly by about 2 °C in the forthcoming 120 minutes. By determining the interval between the occurrences of the temperature change patterns, another estimate of the length of the natural sleep cycle of the person can be calculated.

This additional information may be used for providing additional/alternative estimates on the circadian rhythm, the length of the sleep cycle, and the stages of the natural sleep cycle during the circadian rhythm. Certainty and accuracy of a detection method according to the present disclosure may be improved by combining the detection of stages of sleep and the detection of the temperature patterns. Alternatively, in some embodiments, the length of the sleep cycle may be based solely on the temperature change patterns. For example, the length of sleep cycle in the second embodiment discussed above may be based on the interval between temperature change patterns instead of the intervals between the detected sleep stages. The length of sleep cycle may also be based on the interval between temperature change patterns in the third embodiment above.

The accuracy of the estimate of the circadian rhythm and the length of the sleep cycle may be further improved by gathering data for a plurality of days and by using this data for calculating the estimate. In some embodiments, a method according to the present disclosure may further comprise monitoring movements of the monitored person in order to improve the accuracy of the determination of the sleep cycle and the circadian rhythm.

The present disclosure further discloses a detection unit and a system for detecting stages of sleep of a person. The detection unit may comprise means configured to receive samples of temperature and heart rate of the person, and carry out the steps of an above-described method according to the present disclosure. The detection unit may be a stand-alone apparatus or it may be a part of a larger system.

For example, the detection unit may be a stand-alone apparatus that comprises both sensors for measuring a distal skin temperature and heart rate of a person, and means for implementing the method for detecting stages of sleep of a person according to the present disclosure. The apparatus may comprise control unit comprising a computing device (such as a processor, an FPGA, or an ASIC) and a memory which may act as the means for implementing the method. The stand-alone apparatus may be in the form a wearable device, such as an activity bracelet or a heart rate monitor, that comprises a temperature sensor for monitoring a distal skin temperature of the person. The control unit in the wearable device may be configured to receive samples of the measured temperature (e.g. distal skin temperature) and heart rate from the sensors and detect the stages of sleep based on the samples. The control unit may be configured to calculate percentages or amount (e.g. in minutes) of different stages of sleep based on the sample, and the wearable device may comprise a display on which the calculated percentages may be shown.

The control unit of the wearable device may also be configured to estimate an optimal instant for a wake-up and/or an optimal instant for turning in for sleep on the basis of the detected stages of sleep. The wearable device may implement a timer or clock function, and once an optimal instant has been reached, the wearable device may cause an alarm to be raised, in the form of an audible or visual cue, for example. The wearable device may be configured to implement also other functionalities in addition to a method according to the present disclosure.

In some embodiments, the detection unit may also be a part of a larger detection system. For example, a detection system may comprise a separate measurement unit comprising a distal skin temperature sensor and a heart rate sensor and a separate detection unit that configured to receive samples of distal skin temperature and heart rate from the measurement unit. Alternatively or in addition, the measurement unit may provide the detection unit with temperature variability and/or heart rate variability data calculated on the basis of the distal skin temperature and heart rate data.

The measurement unit may comprise a wireless communication unit through which the temperature and heart rate data is sent to the separate detection unit. The wireless connection unit may transmit the temperature and heart rate data via Bluetooth, ZigBee, near field communication (NFC), or infrared protocols, for example. The measurement unit may be in the form of an activity tracker, a smartwatch, an earbud, a ring, an e-sticker (i.e. an adhesive sensor), for example. The separate detection unit may be a generic computing device or system, for example. A method according to the present disclosure may be implemented in the form of a computer program product having instructions which, when executed by the computing device or system, cause the computing device or system to perform a method according to the present disclosure, also including the first, second, and third embodiment described above. For example, the separate detection unit may be a handheld communication device, such as a smart phone or a tablet computer or even infotainment device or any kind of medical device, to which the computer program product is downloaded. The handheld communication device acting as the separate detection unit may be configured to wirelessly receive temperature and heart rate data and/or temperature variability and heart rate variability data originating from the measurement unit and detect and display sleep quality information, such as information on stages of sleep, on the basis of the data. The communications device may also determine an optimal time window for sleep of the person on the basis of the received data. The handheld communication device may be configured to estimate an optimal instant for a wake-up and/or an optimal instant for turning in for sleep on the basis of the determined optimal time window for sleep, and raise an alarm on the basis of the estimated optimal instant. The handheld-communication device may utilize personal preferences of the user stored in the device in the estimation of the optimal time window for sleep.

Alternatively, cloud computing may be utilized for implementing a method according to the present disclosure, also including the first, second, and third embodiment described above. For example, a cloud computing system acting as a separate detection unit may configured to receive temperature and heart rate data and/or temperature variability and heart rate variability data of a person, detect the stages of sleep and/or temperature change patterns on the basis of the data, and determine sleep quality information and/or an optimal time window for sleep of the person. The cloud computing system may be configured to estimate an optimal instant for a wake-up and/or an optimal instant for turning in for sleep on the basis of the determined optimal time window for sleep, and cause an alarm to be raised on the basis of the estimated optimal instant. For example, the cloud computing system may send an indication to a handheld communication device that an alarm should be raised. The handheld communication device then raises an alarm on the basis of this indication.

Functionalities of the method according to the present disclosure may also be divided between a cloud computing system and a separate decision-making unit. For example, the cloud may detect the stages of sleep and/or temperature change patterns as described above, and send information on the detected stages of sleep and/or temperature change patterns to a decision-making unit that estimates the quality of sleep and/or calculate and/or determines the optimal time window for sleep on the basis of the timing information. The decision-making unit may be a handheld communication device, for example. The decision-making unit may be further configured to calculate an optimal instant for a wake-up and/or an optimal instant for turning in for sleep, and raise an alarm on the basis of the estimated optimal instant based on personal preferences of the user stored on the decision-making unit.

The method (and a detection unit and system implementing the method) according to the present disclosure may be utilized in various applications. For example, in addition to the examples above, the method according to the present disclosure may also be utilized in minimizing jet lag. For a human, it is possible to adjust the circadian rhythm approximately by one hour per day. With the method according to the present disclosure, this adjustment can be done in a systematic manner.

In addition, since muscle recovery after exercise is closely related to the ratio of deep sleep within the total sleep, the information on ratios of different stages of sleep may be utilized when determining an optimal window of sleep for an athlete so s/he will have sufficient deep sleep for muscle recovery, for example. Further, humans need a sufficient time of REM sleep in order to be assimilate cognitive and motoric skills that are very essential skills e.g. for athletes. Recovery algorithms are based on the HRV and they can be significantly improved by using the quantity and quality of sleep. Further, medical personnel may utilize the information when determining the symptoms and cure of a patient in case of depression and sleep apnea, for example.

It is obvious to a person skilled in the art that the method and system according to the present disclosure can be implemented in various ways. The invention and its embodiments are not limited to the examples described above but may vary within the scope of the claims.

## Claims

1. A computer-implemented method for detecting stages of sleep of a person, wherein the method comprises
receiving distal skin temperature data and heart rate data of the person,
determining distal skin temperature variability on the basis of the distal skin temperature data over time and determining heart rate variability (HRV) on the basis of the heart rate data over time,
detecting stages of sleep of the person by
i) detecting that the person is in a REM (Rapid Eye Movement) sleep stage when the distal skin temperature variability is below a first temperature variability limit and the heart rate variability is above a first HRV limit, and/or
ii) detecting that the person is in a deep sleep stage when the distal skin temperature variability is below the first temperature variability limit and the heart rate variability is below a second HRV limit.

2. A method according to claim 1, wherein the method further comprises
calculating ratios of different stages of sleep of the person based on the basis of the detected stages of sleep.

3. A method according to claim 1 or 2, wherein the method further comprises
estimating an optimal instant for a wake-up and/or an optimal instant for turning in for sleep on the basis of the detected stages of sleep.

4. A method according to claim 3, wherein the method comprises
determining a length of a sleep cycle of the person,
calculating a number of specific sleep stages already observed during night's sleep on the basis of the detected stages of sleep, and
determining the optimal wake-up instant on the basis of the length of the sleep cycle and the number of sleep stages already observed so that the person will have slept a desired number of sleep cycles at the optimal wake-up instant.

5. A method according to claim 3, wherein the method comprises
determining a length of a sleep cycle of the person,
determining a fixed reference point within a circadian rhythm of the person,
determining the optimal wake-up instant on the basis of the length of the sleep cycle and the fixed reference point so that the person will have slept a desired number of sleep cycles at the optimal wake-up instant.

6. A method according to any one of claims 1 to 5, wherein the method further comprises
measuring the distal skin temperature data and the heart rate data of the person with a measurement band or bracelet worn by the person.

7. A method according to claim 5, wherein the determining of the reference point of a circadian comprises
detecting a temperature change pattern in the samples of the distal skin temperature data, wherein the temperature change pattern indicates a reference point for an optimal time window for sleep, wherein the temperature change pattern is in the form of a drop in the distal skin temperature followed by an increase in the distal skin temperature, where the drop and the increase occur within a time window of ten minutes or less.

8. A method according to claim 7, wherein a magnitude of the drop is approximately 0.5 °C and a magnitude of the increase is approximately 1.5 °C.

9. A detection unit comprising means configured to carry out a method according to any one of claims 1 to 8.

10. A detection unit according to claim 9, wherein the detection unit is a wearable device in the form of band or bracelet comprising
a temperature sensor,
a heart rate sensor, and
a control unit configured to carry out a method according to any one of claims 1 to 8.

## Patentansprüche

1. Computerimplementiertes Verfahren zum Erfassen von Schlafphasen einer Person, wobei das Verfahren Folgendes umfasst:
Empfangen von Daten der distalen Hauttemperatur und Herzfrequenzdaten der Person,
Bestimmen der Variabilität der distalen Hauttemperatur auf Basis der Daten der distalen Hauttemperatur im Laufe der Zeit und Bestimmen der Herzfrequenzvariabilität (HRV) auf Basis der Herzfrequenzdaten im Laufe der Zeit,
Erfassen von Schlafphasen der Person durch
i) Erfassen, dass sich die Person in einer REM(Rapid Eye Movement)-Schlafphase befindet, wenn die Variabilität der distalen Hauttemperatur unterhalb einer ersten Temperaturvariabilitätsgrenze und die Herzfrequenzvariabilität oberhalb einer ersten HRV-Grenze liegt, und/oder
ii) Erfassen, dass sich die Person in einer Tiefschlafphase befindet, wenn die Variabilität der distalen Hauttemperatur unterhalb der ersten Temperaturvariabilitätsgrenze und die Herzfrequenzvariabilität oberhalb einer zweiten HRV-Grenze liegt.

2. Verfahren nach Anspruch 1, wobei das Verfahren ferner Folgendes umfasst:
Berechnen der Verhältnisse der verschiedenen Schlafphasen der Person basierend auf Basis der erfassten Schlafphasen.

3. Verfahren nach Anspruch 1 oder 2, wobei das Verfahren ferner Folgendes umfasst:
Schätzen eines optimalen Zeitpunkts für das Aufwachen und/oder eines optimalen Zeitpunkts für das Einschlafen auf Basis der erfassten Schlafphasen.

4. Verfahren nach Anspruch 3, wobei das Verfahren Folgendes umfasst:
Bestimmen einer Länge eines Schlafzyklus der Person,
Berechnen einer Anzahl spezifischer Schlafphasen, die bereits während des Nachtschlafs beobachtet wurden, auf Basis der erfassten Schlafphasen, und
Bestimmen des optimalen Aufwachzeitpunkts auf Basis der Länge des Schlafzyklus und der Anzahl an Schlafphasen, die bereits beobachtet wurden, so dass die Person eine gewünschte Anzahl an Schlafzyklen zum optimalen Aufwachzeitpunkt geschlafen haben wird.

5. Verfahren nach Anspruch 3, wobei das Verfahren Folgendes umfasst:
Bestimmen einer Länge eines Schlafzyklus der Person,
Bestimmen eines festen Bezugspunkts innerhalb eines zirkadianen Rhythmus der Person,
Bestimmen des optimalen Aufwachzeitpunkts auf Basis der Länge des Schlafzyklus und des festen Bezugspunkts, so dass die Person eine gewünschte Anzahl an Schlafzyklen zum optimalen Aufwachzeitpunkt geschlafen haben wird.

6. Verfahren nach einem der Ansprüche 1 bis 5, wobei das Verfahren ferner Folgendes umfasst:
Messen der Daten der distalen Hauttemperatur und der Herzfrequenzdaten der Person mit einem Messband oder - armband, das von der Person getragen wird.

7. Verfahren nach Anspruch 5, wobei das Bestimmen des Bezugspunkts eines zirkadianen Rhythmus das
Erfassen eines Temperaturänderungsmusters in den Proben der Daten der distalen Hauttemperatur umfasst, wobei das Temperaturänderungsmuster einen Bezugspunkt für ein optimales Zeitfenster für Schlaf angibt, wobei das Temperaturänderungsmuster die Form eines Abfalls der distalen Haupttemperatur gefolgt von einem Anstieg der distalen Hauttemperatur hat, wobei der Abfall und der Anstieg innerhalb eines Zeitfensters von zehn Minuten oder weniger auftreten.

8. Verfahren nach Anspruch 7, wobei der Umfang des Abfalls ungefähr 0,5 °C beträgt und der Umfang des Anstiegs ungefähr 1,5 °C beträgt.

9. Erfassungseinheit, die ein Mittel umfasst, das dazu konfiguriert ist, ein Verfahren nach einem der Ansprüche 1 bis 8 auszuführen.

10. Erfassungseinheit nach Anspruch 9, wobei die Erfassungseinheit eine tragbare Vorrichtung in Form eines Bands oder eines Armbands ist, das Folgendes umfasst:
einen Temperatursensor,
einen Herzfrequenzsensor, und
eine Steuereinheit, die dazu konfiguriert ist, ein Verfahren nach einem der Ansprüche 1 bis 8 auszuführen.

## Revendications

1. Procédé mis en œuvre par ordinateur pour la détection de stades de sommeil d'une personne, ce procédé comprenant
la réception de données de température cutanée distale et de données de fréquence cardiaque de la personne,
la détermination de la variabilité de température cutanée distale sur la base des données de température cutanée distale dans le temps et la détermination de la variabilité de fréquence cardiaque (HRV) dans le temps,
la détection de stades de sommeil de la personne en
i) détectant que la personne est dans un stade de sommeil REM (à mouvement rapide des yeux) lorsque la variabilité de température cutanée distale est en dessous d'une première limite de variabilité de température et la variabilité de fréquence cardiaque est au-dessus d'une première limite de HRV, et/ou
ii) détectant que la personne est dans un stade de sommeil profond lorsque la variabilité de température cutanée distale est en dessous de la première limite de variabilité de température et la variabilité de fréquence cardiaque est au-dessus d'une seconde limite de HRV.

2. Procédé selon la revendication 1, ce procédé comprenant en outre
le calcul de rapports de différents stades de sommeil de la personne en se basant sur la base des stades détectés de sommeil.

3. Procédé selon la revendication 1 ou 2, ce procédé comprenant en outre
l'estimation d'un instant optimal pour un réveil et/ou d'un instant optimal pour s'endormir sur la base des stades détectés de sommeil.

4. Procédé selon la revendication 3, ce procédé comprenant
la détermination d'une longueur d'un cycle de sommeil de la personne,
le calcul d'un nombre de stades de sommeil spécifiques déjà observés pendant le sommeil nocturne sur la base des stades détectés de sommeil, et
la détermination de l'instant de réveil optimal sur la base de la longueur du cycle de sommeil et du nombre de stades de sommeil déjà observés de manière à ce que la personne ait dormi pendant un nombre désiré de cycles de sommeil à l'instant de réveil optimal.

5. Procédé selon la revendication 3, ce procédé comprenant
la détermination d'une longueur d'un cycle de sommeil de la personne,
la détermination d'un point de référence fixe dans un rythme circadien de la personne,
la détermination de l'instant de réveil optimal sur la base de la longueur du cycle de sommeil et du point de référence fixe de manière à ce que la personne ait dormi pendant un nombre désiré de cycles de sommeil à l'instant de réveil optimal.

6. Procédé selon l'une quelconque des revendications 1 à 5, ce procédé comprenant en outre
la mesure des données de température cutanée distale et des données de fréquence cardiaque de la personne avec une bande de mesure portée par la personne.

7. Procédé selon la revendication 5, dans lequel la détermination du point de référence d'un rythme circadien comprend
la détection d'un modèle de changement de température dans les échantillons des données de température cutanée distale, le modèle de changement de température indiquant un point de référence pour une fenêtre temporelle optimale pour le sommeil, le modèle de changement de température étant sous la forme d'une chute de la température cutanée distale suivie d'une augmentation de la température cutanée distale, la chute et l'augmentation se produisant pendant une fenêtre temporelle de dix minutes ou moins.

8. Procédé selon la revendication 7, dans lequel une amplitude de la chute est approximativement de 0,5 °C et une amplitude de l'augmentation est approximativement de 1,5 °C.

9. Unité de détection comprenant un moyen configuré pour exécuter un procédé selon l'une quelconque des revendications 1 à 8.

10. Unité de détection selon la revendication 9, l'unité de détection étant un dispositif portable sous la forme d'une bande ou d'un bracelet comprenant
un capteur de température ;
un capteur de fréquence cardiaque ; et
une unité de commande configurée pour exécuter un procédé selon l'une quelconque des revendications 1 à 8.
